# FASCICULE DE BREVET EUROPEEN

(11) **EP 1 652 435 B1**
(45) Date de publication et mention de la délivrance du brevet: **14.06.2017**
(21) Numéro de dépôt: 05291997.4
(22) Date de dépôt: 27.09.2005
(51) Int. Cl.: A23L 33/105, A23L 33/10

(54) **Utilisation d'algues rouges soumises à des conditions extrêmes de température et de luminosité**
Verwendung von roten Algen, die extremen Temperatur- und Lichtbedingungen ausgesetzt sind
Use of red algae subjected to extreme temperature and light conditions.

(30) Priorité: 01.10.2004 FR 0410416
(43) Date de publication de la demande: 03.05.2006
(73) Titulaire: EXSYMOL S.A.M., 98000 Monte Carlo (MC)
(72) Inventeur: Seguin, Marie-Christine, 98000 Monaco (MC); Nicolay, Jean-Francois, 06000 Nice (FR)
(74) Mandataire: Nevant, Marc

(56) Documents cités:
- EP-A- 1 325 735
- DE-A1- 2 651 617
- FR-A- 2 655 268
- FR-A- 2 832 628
- FR-A- 2 832 629
- US-A- 4 897 266
- US-A1- 2003 190 327
- LAYCOCK M V ET AL: "THE OCCURRENCE AND SEASONAL VARIATION OF GIGARTININE AND L-CITRULLINYL-L-ARGININE IN CHONDRUS CRISPUS STACKH" CANADIAN JOURNAL OF BIOCHEMISTRY, NATIONAL RESEARCH COUNCIL OF CANADA, OTTAWA,, CA, vol. 55, no. 1, janvier 1997 (1997-01), pages 27-30, XP009054912 ISSN: 0008-4018
- ITO K ET AL: "AMINO-ACID COMPOSITION OF THE ETHANOLIC EXTRACTIVES FROM 31 SPECIES OF MARINE RED ALGAE" 1977, BIOSIS , XP002247061 * le document en entier *
- JIMENEZ-ESCRIG A ET AL: "Antioxidant activity of fresh and processed edible seaweeds." JOURNAL OF THE SCIENCE OF FOOD AND AGRICULTURE, vol. 81, no. 5, 2001, page 530-534, XP002361824 Spain

## Description

L'invention concerne l'utilisation de macroalgues rouges soumises à des conditions extrêmes de température et de luminosité, ou d'extraits issus de telles algues, comme antioxydant.

L'invention trouve notamment application dans les domaines nutritionnel, diététique et nutraceutique.

La valeur alimentaire des algues est connue de longue date puisque, depuis des siècles, les populations de pays du Sud-Est asiatique en consomment (Nisizawa K.et al., Hydrobiol. (1987), vol.151/1542, pp.5-29), notamment pour leurs saveurs et souvent comme "sea vegetable" ou légume d'accompagnement issu de la mer. Dans les pays occidentaux, les algues sont essentiellement utilisées comme source de phycocolloïdes, principalement pour l'obtention d'alginates, de carraghénanes ou d'agars aux propriétés épaississantes, gélifiantes ou stabilisantes largement reconnues (Mc Huhh D., Hydrobiol. (1991), vol.221, ppm.19-29). L'intérêt nutritionnel des algues est cependant bien établi avec une richesse à la fois en minéraux, fibres, protéines, acides aminés essentiels et vitamines (Burtin P., Electron. J. Environ. Agric. Food Chem. (2003), vol.2(4) ISSN : 1579-4377 ; Jimenez-Escrig et al., Arch. Latinoam. de Nutr. (1999), vol.49, pp.114-120 ; Carbini L. et al., Riv. Sci. Aliment. (1998), vol.27, pp. 169-173).

Par ailleurs aujourd'hui, il est admis qu'il nous faut lutter au mieux, dans notre vie quotidienne, contre les facteurs environnementaux qui induisent un stress oxydant (UV, pollution, etc) à effet délétère sur notre organisme, tel une trop grande production de radicaux libres ou d'espèces réactives oxygénées. Une supplémentation en antioxydants constitue de ce fait actuellement l'une des plus vives recommandations nutritionnelles ("Des antioxydants pour nous aider à vieillir en bonne santé", Nutranews, Décembre 2001). C'est en outre une des principales revendications des fabricants de compléments ou suppléments alimentaires, ou d'alicaments dans l'industrie nutraceutique, avec la mise sur le marché de produits aux multiples vertus, notamment antioxydantes, les "multi-nutriments" ("Les multi-nutriments, des suppléments nutritionnels essentiels", Nutranews, Avril 2003).

Le potentiel antioxydant des algues est largement documenté dans l'état de la technique. L'activité antioxydante de macroalgues fraîches ou séchées fait ainsi l'objet d'une récente étude (Jimenez-Escrig A. et al., J. Sci. Food Agric. (2001), vol.81, pp.530-534) où il est conclu, d'une part, à une corrélation entre le pouvoir antioxydant et la richesse de l'algue en substances polyphénoliques que sont les phloroglucinols, et, d'autre part, au potentiel antioxydant des algues brunes supérieur à celui des algues rouges. Il est à noter dans cet article, pour l'espèce rhodophyte *Chondrus crispus* de qualité usuelle (ou standard), la non détection d'une activité antioxydante.

La demande de brevet FR-A-2 655 268 décrit l'utilisation d'extraits d'algues brunes, rouges ou vertes pour la préparation de compositions, notamment alimentaires, à activité anti-radicalaire. Il est mentionné que les algues brunes présentent l'activité anti-radicalaire la plus importante. Nulle information n'est divulguée quant à la nature des substances responsables de cette activité d'origine algale. La demande de brevet FR-A-2 832 629 décrit l'utilisation de macroalgues rouges comme antioxydant dans les domaines nutritionnel, diététique et nutraceutique.

Par ailleurs, il est à souligner que les algues rouges de qualité usuelle, généralement exploitées pour leur richesse en phycocolloïdes, sont récoltées ordinairement et classiquement en phase de prolifération. Le métabolisme qu'elles présentent est donc tout à fait différent de celui de l'algue encore au stade végétatif. Par exemple, dans le cas particulier de l'algue *Chondrus crispus,* un examen de sa composition révèle une consommation très rapide d'un dipeptide accumulé en phase végétative, le dipeptide citrullinylarginine, dès le retour du printemps et de conditions favorables à la croissance de la plante (Laycock M.V. et al., Can J. Biochem. (1981), vol.59, pp.522-527). D'une manière générale, il est observé, lors de la croissance d'une algue, à la fois une augmentation de la teneur en phycocolloides et une diminution de celle en composés à valeur énergétique (protéines, acides aminés, etc.) puisque ces derniers sont activement consommés. Dans le cas de l'algue *Chondrus crispus,* ceci pourrait expliquer la faible valeur nutritionnelle accordée par les auteurs en conclusion d'une étude sur l'effet de régimes algaux sur la croissance de mollusques marins (Mai K. et al., Aquaculture (1994), vol.128, pp.115-130).

La littérature rapporte que certaines macroalgues rouges peuvent, lors de conditions hivernales avec une température de l'eau basse et un minimum d'intensité lumineuse, capter l'azote (ions nitrate, nitrite, ammonium) des eaux côtières et le stocker sous forme de petits peptides ou/et divers aminoacides libres (gigartinine, citrulline, arginine, ornithine, etc) variables selon l'espèce.

Lesdites algues rouges naturellement identifiées avec un tel profil au sortir de l'hiver ont été successivement *Grateloupia turuturu* (Miyazawa K. et al., Bulletin of the Japonese Society of Scientific Fisheries (1974), vol.40, pp.815-818), *Ahnfeltia plicata, Gracilaria sp., Petrocelis middendorfii, Polyides rotundus, Polysiphonia lanosa, Rhodomela confervoides* (Laycock M.V. et al., Can J. Biochem. (1977), vol.55, pp.27-30) et *Chondrus crispus* (Laycock M.V. et al., Can J. Biochem. (1981), vol.59, pp.522-527).

Cette modification métabolique au sein de l'algue a été exploitée pour des applications en dermatologie. Ainsi, la demande de brevet européen EP 1 350 517 A1 (déposée par la demanderesse) décrit l'utilisation dermatologique, comme agents de soin et de traitement pour la peau et de phanères, de molécules chimiques analogues du dipeptide citrullinylarginine et d'un extrait enrichi en polypeptides contenant ce dipeptide.

En poursuivant ses recherches, la demanderesse a constaté que les macroalgues rouges soumises à des conditions extrêmes de température et de luminosité, ainsi que certaines fractions issues de l'extraction de telles algues, présentent un fort caractère antioxydant, et ce, de manière inattendue au regard d'une comparaison avec les mêmes algues rouges mais récoltées en pleine croissance (dites "algues standards") ou pour les fractions obtenues à partir desdites algues.

La demanderesse a noté en outre, lors d'études comparatives avec des antioxydants de référence et une réplique chimique du dipeptide citrullinylarginine, que ce caractère antioxydant ne pouvait être expliqué par la seule accumulation du dipeptide dans l'algue soumise à des conditions extrêmes de température et de luminosité. Elle en a conclu que d'autres constituants accumulés dans cette qualité d'algue lors de la période hivernale contribuent également au pouvoir antioxydant, et a identifié certains de ces constituants.

Ainsi, selon un premier aspect, l'invention a pour objet l'utilisation nutritionnelle, diététique ou nutraceutique, de macroalgues rouges soumises à des conditions extrêmes de température et de luminosité, ou d'extraits issus de telles algues, comme agent antioxydant.

Les macroalgues rouges sont choisies parmi les espèces *Grateloupia turuturu, Ahnfeltia plicata, Gracilaria sp., Petrocelis middendorfii, Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* et *Chondrus crispus,* cette dernière espèce étant particulièrement préférée.

Par "macroalgues rouges soumises à des conditions extrêmes de température et de luminosité", il faut comprendre des algues au stade végétatif qui ont été exposées à une eau froide et une luminosité réduite permettant la formation de constituants antioxydants, mais aussi énergétiques. Ces conditions particulières peuvent être soit des conditions naturelles hivernales, soit des conditions artificielles avec un procédé de culture en bassin reproduisant les conditions naturelles ci-dessus. Les paramètres "sensibles" pour de telles conditions artificielles sont notamment décrites pour l'espèce *Chondrus crispus* (Laycock M.V. et al., Can J. Biochem. (1981), vol.59, pp.522-527).

Quel que soit l'environnement "hivernal" retenu, naturel ou reproduit, une caractéristique importante de l'invention est que la qualité d'algue recherchée n'est obtenue que lorsque l'algue est exposée aux conditions suivantes :
- une étape de récolte de l'espèce algale à la fin de la saison hivernale (hiver septentrional), de préférence entre Mars et début Avril;
- une "exposition" de l'espèce algale à une eau froide comprise entre 0 et 8°C, de préférence entre 0 et 4°C;
- une "exposition" de l'espèce algale à une intensité lumineuse comprise entre 40 et 120 langley.day⁻¹ PAR;
- une "exposition" de l'espèce algale à une eau contenant des ions nitrate et ammonium, de préférence une eau contenant des ions nitrate à la teneur de 1 à 6 µmole;
- une "exposition" de l'espèce algale à une eau de salinité comprise entre 10 et 30 pour mille.

Ces conditions sont celles que l'on trouve dans des zones subarctiques (au nord du 45° parallèle N). Les côtes de la Nouvelle Ecosse au Canada constituent par exemple un biotope particulièrement favorable à l'obtention de la qualité d'algue recherchée.

Selon un mode de réalisation préféré de l'invention, une biomasse "uni-algale" d'algue rouge, par exemple de *Chondrus crispus,* est tout d'abord obtenue après une sélection rigoureuse d'une qualité d'algue dépourvue de toute algue "parasite". Elle est ensuite placée en bassin (fin automne/début d'hiver) puis maintenue en eau de mer filtrée pendant tout l'hiver, sans agitation ni oxygénation, dans les conditions indiquées ci-dessus. Elle est alors récoltée en fin de période végétative (Mars/Avril), avant que l'algue ne passe en phase proliférative. Cette algue est finalement soigneusement lavée, puis séchée.

L'algue "exposée" est caractérisée par une teneur élevée en constituants antioxydants et énergétiques, et elle comprend notamment :
- le dipeptide citrullinylarginine,
- les aminoacides libres taurine et citrulline et/ou leurs dérivés,
- des aminoacides "mycosporine-like",
- les ions zinc et/ou sélénium,
- le floridoside.

Les dérivés de la taurine peuvent être la N-diméthyl-taurine et la N-triméthyltaurine, et ceux de la citrulline les aminoacides lividine, grateloupine, gigartinine et gongrine.

Préférentiellement, l'algue "exposée" comprend :
- de 2 à 10 % de citrullinylarginine en poids de matière sèche,
- de 6 à 30 mg de taurine dans 1 g d'algue sèche,
- de 6 à 30 mg de citrulline dans 1 g d'algue sèche,
- de 0,7 à 1,5 mg d'aminoacides "mycosporine-like" dans 1 g d'algue sèche,
- de 20 à 80 ppm de zinc et de 5 à 40 ppm de sélénium,
- de 3 à 6 % de floridoside en poids de matière sèche,
alors que sa teneur en azote, d'environ 4%, est indicative d'une teneur en protéines/peptides/aminoacides libres tous confondus.

Les macroalgues rouges soumises à des conditions extrêmes de température et de luminosité sont donc caractérisées par une teneur en protéines ou en peptides plus importante que les macroalgues rouges standards. Dans les algues soumises à des conditions extrêmes de température et de luminosité :
- le dipeptide citrullinylarginine est ainsi particulièrement intéressant, car il constitue une source d'arginine, aminoacide semi-essentiel décrit pour participer au métabolisme énergétique de tissus sous forme de phosphoarginine et constituer un support à la glycolyse (Gade G. et al., Comp. Biochem. Physiol. (1986), vol.83B, pp.255-272);
- la citrulline, issue du dipeptide ou celle libre accumulée dans l'algue, est susceptible de représenter elle aussi une valeur énergétique intéressante puisque c'est un précurseur de l'ornithine, de l'acide glutamique et de la proline;
- la taurine possède des propriétés antioxydantes intéressantes contre le phénomène de péroxydation lipidique via une affinité pour les phospholipides membranaires et une stabilisation des membranes cellulaires (Birdsall T.C., Alternative Med. Rev. (1998), vol.3, pp.128-136); la taurine est communément utilisée en nutrition puisqu'une supplémentation en cet aminoacide constitue un apport énergétique dont l'origine serait également une capacité à moduler la glycolyse (Redmond H.P et al., Nutrition, (1998), vol.14, 599-604) et ((J.B. Lombardini et al., Ed Plenum Press, N.Y. (1992));
- les aminoacides "mycosporine-like", tels la palythine, la shinorine et l'astérina, ont des propriétés protectrices contre le rayonnement ultra-violet démontrées (Bandaranayake W.M., Nat. Prod. Rep. (1998), vol.15, pp.159-172);
- les ions zinc sont connus pour leurs propriétés antioxydantes (Zago M.P., Free Radic. Biol. Med. (2001), vol.31, pp.266-274), et le sélénium est un cofacteur nécessaire à l'activité de certaines enzymes antioxydantes (Tappel A.L., Curr. Top. Cell. Regul. (1984), vol.24, pp.87);
- le 2-glycérol-galactopyranoside ou floridoside est un monosaccharide anti-stress (Simon-Colin C., J. Appl. Phycol. (2002), vol.14, pp.123-127).

Par ailleurs, il est évident que d'autres constituants communément identifiés dans les macroalgues rouges (sels minéraux, fibres, etc) contribuent eux aussi à l'intérêt nutritionnel des algues soumises à des conditions extrêmes de température et de luminosité.

L'invention concerne également les extraits, notamment les extraits alcooliques, hydroalcooliques ou hydroglycoliques, de ces macroalgues rouges exposées à des conditions extrêmes de température et de luminosité. Ces extraits sont obtenus selon des techniques bien connues de l'homme du métier, et comprennent les mêmes constituants antioxydants et énergétiques que ceux des macroalgues rouges "exposées", préférentiellement dans les quantités mentionnées ci-dessous :
- au plus 15 % de citrullinylarginine en poids d'extrait,
- au plus 45 mg de taurine dans 1 g d'extrait,
- au plus 45 mg de citrulline dans 1 g d'extrait,
- au plus 2,25 mg d'aminoacides "mycosporine-like" dans 1 g d'extrait,
- au plus 120 ppm de zinc et au plus 60 ppm de sélénium,
- au plus 9 % de floridoside en poids d'extrait.

Les macroalgues rouges "exposées" selon l'invention, ou les extraits de telles algues, sont avantageusement utilisé(e)s comme ingrédient actif pour la préparation de compositions à fonction nutritive et antioxydante, telles des compositions nutritionnelles, diététiques ou nutraceutiques.

Ainsi, selon un autre aspect, l'invention concerne une composition nutritionnelle, diététique ou nutraceutique contenant une ou plusieurs espèces de macroalgues rouges soumises à des conditions extrêmes de température et de luminosité (ou un ou plusieurs extraits de ces algues). Généralement, les macroalgues rouges ou leurs extraits sont transformé(e)s en une poudre micronisée ou une pâte, puis mélangé(e)s avec les excipients couramment utilisés en nutrition, diététique ou nutraceutique pour former ladite composition. La composition de l'invention peut également contenir d'autres ingrédients actifs selon les besoins du domaine considéré. Elle peut être conditionnée en gélules, comprimés, sachets, etc.

L'invention est illustrée par les exemples et compositions suivantes, donnés à titre purement indicatif.

### Exemple 1 : détermination de l'activité antioxydante d'une fraction éthanolique issue de Chondrus crispus "exposé"

On a récolté des algues de l'espèce *Chondrus crispus* soumises à des conditions extrêmes de température et de luminosité selon le mode opératoire indiqué ci-dessus, puis on les a lavées et séchées. Il a été obtenu une fraction éthanolique selon le protocole d'extraction suivant : l'algue broyée est tout d'abord humidifiée par de l'eau à température ambiante. Elle est alors l'objet d'une extraction directe à l'aide d'éthanol ammoniacal sous agitation à température ambiante. Après filtration, l'extrait est successivement concentré, ajusté avec de l'eau, décoloré sur charbon actif, filtré, et à nouveau concentré.

L'extrait ainsi obtenu contient notamment de la citrullinylarginine, de la taurine et ses dérivés méthylés, des acides gras saturés, des monosaccharides, du floridoside, des aminoacides "mycosporine-like", de la citrulline, des sels minéraux tels que le zinc et du sélénium.

### i) mesure de l'activité piégeur du radical hydroxyle (OH°)

La méthode, décrite par Rehman, A. et coll. (British J. Pharmacol. (1997), vol.122, pp.1702-1706), est utilisée pour la mesure de la constante de vitesse de piégeage du radical hydroxyle Ks(OH°)

L'extrait selon l'invention est comparé au mannitol (piégeur de référence), au dipeptide citrullinylarginine (produit de synthèse) et à l'acide ascorbique (piégeur de référence), dont le Ks(OH°) est mesuré selon Cabelli D.E., J. Phys. Chem. (1983), vol.87, pp.1809-1812). Le tableau 1 rassemble les valeurs moyennes obtenues à partir de 4 expérimentations indépendantes.

**Tableau 1**

| Antioxydant testé | Ks(OH°) (10⁹.M⁻¹.s⁻¹) |
|---|---|
| Mannitol | 2,39 ± 0,38 |
| Citrullinylarginine | 5,16 ± 0,78 |
| Extrait éthanolique | 6,01 ± 0,44 |
| Acide ascorbique | 10,1 ± 2,3 |

### ii) mesure du pouvoir antioxydant "global"

Le système de production d'espèces réactives dérivées de l'oxygène xanthine oxydase / hypoxanthine + EDTA, décrit par Nowak D. et coll. (Biomed. Biochem. Acta (1991), vol.50, pp.265-272), a été utilisé pour déterminer le pouvoir antioxydant d'une fraction éthanolique issue de *Chondrus crispus* "exposé", Ce pouvoir antioxydant, caractérisé par la somme des effets contre l'anion superoxyde (O₂°⁻), le peroxyde d'hydrogène (H₂O₂ et OH°, est exprimé par un pourcentage de protection d'une molécule "détectrice", le désoxyribose (tableau 2).

**Tableau 2**

| Antioxydant testé | % de protection |
|---|---|
| Extrait éthanolique (avec ajustement à 1mM en taurine et dérivés) | 19,5 ± 2,5 |
| Taurine (1 mM) | 8,7 ± 2 |
| Mannitol (1 mM) | 21,1 ± 2 |

### Exemple 2 : détermination de l'activité antioxydante d'une fraction hydroalcoolique issue de Chondrus crispus "exposé"

On a récolté des algues de l'espèce *Chondrus crispus* soumises à des conditions extrêmes de température et de luminosité selon le mode opératoire indiqué ci-dessus, puis on les a lavées et séchées. Il a été obtenu une fraction hydroéthanolique (80 EtOH/20 H₂O) selon le protocole d'extraction suivant : l'algue broyée est tout d'abord humidifiée par de l'eau à température ambiante. Elle est alors l'objet d'une extraction directe à l'aide d'une solution hydroéthanolique ammoniacale sous agitation à température ambiante. Après filtration, l'extrait est successivement concentré, ajusté avec de l'eau, décoloré sur charbon actif, filtré, et à nouveau concentré.

L'extrait ainsi obtenu contient notamment de la citrullinylarginine, de la taurine, des aminoacides "mycosporine-like", de la L-citrulline, des sels minéraux tels que du zinc, du floridoside et du sélénium.

### i) mesure de l'activité piégeur du radical hydroxyle (OH°)

Le protocole utilisé est celui décrit à l'exemple 1. Les résultats sont rassemblés dans le tableau 3.

**Tableau 3**

| Antioxydant testé | Ks(OH°) (10⁹.M⁻¹.s⁻¹) |
|---|---|
| Mannitol | 2,39 ± 0,38 |
| Citrullinylarginine | 5,16 ± 0,78 |
| Extrait hydroéthanolique | 10,13 ± 0,91 |
| Acide ascorbique | 10,1 ± 2,3 |

### ii) mesure du pouvoir antioxydant "global"

Le protocole utilisé est celui décrit à l'exemple 1. L'extrait selon l'invention est comparé au dipeptide citrullinylarginine et au mannitol. Les résultats sont rassemblés dans le tableau 4.

**Tableau 4**

| Antioxydant testé | % de protection |
|---|---|
| Extrait hydroéthanolique (avec ajustement à 1mM en citrullinylarginine) | 29,5 ± 2,5 |
| Citrullinylarginine (1 mM) | 5,3 ± 2 |
| Mannitol (1 mM) | 21,1 ± 2 |

### Exemple 3 : détermination et comparaison des activités antioxydantes d'extraits hydroalcooliques de Chondrus crispus "exposé" et "standard"

Les extraits algaux hydroalcooliques de qualité "exposé" et "standard" ont été obtenus selon un même protocole d'extraction. La détermination de leur pouvoir antioxydant a ensuite été réalisée à l'aide du modèle d'oxydation général décrit par Nowack et coll., utilisant le système de production d'espèces réactives xanthine oxydase/hypoxanthine (Biomed. Biochem. Acta (1991), vol 50, pp.265-272).

Comme dans les exemples précédents 1 et 2, le pouvoir antioxydant est exprimé par un pourcentage de protection du désoxyribose (ou pourcentage d'inhibition d'oxydation du désoxyribose). Une variante, consistant à ne pas ajouter d'EDTA, a été introduite afin de mieux distinguer l'effet de composés chélatants contenus dans les extraits, et leur contribution à l'effet antioxydant global.

Les résultats ci-dessous, obtenus à partir de trois essais indépendants, permettent une comparaison du potentiel antioxydant entre les extraits "exposé" et "standard" de Chondrus crispus, à différentes concentrations.

**Tableau 5 (en présence d'EDTA)**

| | % INHIBITION | |
|---|---|---|
| % extrait | Extrait "exposé" | Extrait "standard" |
| 1 | 29,2 | 3,0 |
| 2 | 42,4 | 6,8 |
| 5 | 67,3 | 14,5 |

**Tableau 6 (en absence d'EDTA)**

| | % INHIBITION | |
|---|---|---|
| % extrait | Extrait "exposé" | Extrait "standard" |
| 0,1 | 30,7 | 10,1 |
| 0,25 | 39,7 | 16,3 |

Les résultats regroupés aux tableaux 5 et 6 montrent que les extraits "exposés" utilisés dans le cadre de la présente invention présentent systématiquement une activité anti-oxydante très largement supérieure à celle des extraits "standards".

### Exemple 4 : teneurs en azote total et profils en "acides aminés" des algues de type Chondrus crispus "exposé" et "standard"

| constituant | algue "exposée" | algue 'standard" |
|---|---|---|
| azote total (%) | 4,8 | 2 |
| citrullinylarginine (%) | 4,3 | < 0,01 |
| taurine (%) | 0,54 | < 0,01 |
| citrulline (%) | 0,52 | < 0,01 |

Les premiers résultats d'études in vivo menées sur l'homme sont également en mesure d'illustrer l'utilisation desdites algues aux fins de la présente invention.

On donnera maintenant des exemples de compositions nutritionnelle, diététique ou nutraceutique à haut potentiel antioxydant et haute valeur énergétique conformes à l'invention.

### Composition 1 (présentation en gélule)

- poudre micronisée de *Chondrus crispus* et/ou de *Gymnogongrus devoniensis* (200 mg/gélule)
- excipients qsp 1 gélule en gélatine : cellulose microcristalline, stéarate de magnésium.

### Composition 2 (présentation en gélule)

- extrait sec de *Chondruscrispuset*/*ou* de *Gymnogongrus devoniensis* (150 mg/gélule)
- excipients qsp 1 gélule en gélatine : cellulose microcristalline, amidon, stéarate de magnésium.

### Composition 3 (présentation en comprimé)

- poudre micronisée de *Chondrus crispus* et/ou de *Gymnogongrus devoniensis* (250 mg/comprimé)
- excipients qsp 1 comprimé : cellulose microcristalline, carboxyméthyl-cellulose sodique, silice colloïdale, stéarate de magnésium.

## Revendications

1. Utilisation pour la fabrication d'une composition nutritionnelle, diététique ou nutraceutique, de macroalgues rouges soumises à des conditions extrêmes de température et de luminosité puis récoltées à la fin de l'hiver septentrional, de préférence entre Mars et début Avril, ou d'extraits alcooliques, hydroalcooliques ou hydroglycoliques issus de telles algues, comme agent antioxydant ;
lesdites conditions extrêmes consistant en une exposition à une eau froide comprise entre 0 et 8°C, de salinité comprise entre 10 et 30 pour mille, et contenant des ions nitrate et ammonium, et à une intensité lumineuse comprise entre 40 et 120 langley.day⁻¹ PAR;
lesdites macroalgues rouges étant choisies parmi les espèces *Grateloupia turuturu, Ahnfeltia plicata, G racilaria sp., Petrocelis middendorfii, Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* et *Chondrus crispus;*
lesdites macroalgues rouges ou leurs extraits comprenant les constituants suivants :
- le dipeptide citrullinylarginine,
- les aminoacides libres taurine et citrulline et/ou leurs dérivés,
- des aminoacides "mycosporine-like",
- les ions zinc et/ou sélénium,
- le floridoside.

2. Utilisation selon la revendication 1, **caractérisée en ce que** les macroalgues rouges sont choisies parmi les espèces *Grateloupia turuturu, Ahnfeltia plicata, Gracilaria sp., Petrocelis middendorfii, Polyides rotundus, Polysiphonia lanosa, Rhodomela confervoides, Gymnogongrus devoniensis,* et *Chondrus crispus.*

3. Utilisation selon la revendication 1 ou 2, **caractérisée en ce que** les macroalgues rouges sont de l'espèce *Chondrus crispus.*

4. Utilisation selon l'une des revendications 1 à 3, **caractérisée en ce que** les conditions extrêmes de température et de luminosité consistent en une exposition à une eau froide comprise entre 0 et 4°C, et contenant des ions nitrate à la teneur de 1 à 6 µmole et ammonium.

5. Utilisation selon l'une des revendications 1 à 4, **caractérisée en ce que** lesdites macroalgues comprennent :
- de 2 à 10 % de citrullinylarginine en poids de matière sèche,
- de 6 à 30 mg de taurine dans 1 g d'algue sèche,
- de 6 à 30 mg de citrulline dans 1 g d'algue sèche,
- de 0,7 à 1,5 mg d'aminoacides "mycosporine-like" dans 1 g d'algue sèche,
- de 20 à 80 ppm de zinc et de 5 à 40 ppm de sélénium,
- de 3 à 6 % de floridoside en poids de matière sèche.

6. Utilisation selon l'une des revendications 1 à 4, **caractérisée en ce que** lesdits extraits comprennent :
- au plus 15 % de citrullinylarginine en poids d'extrait,
- au plus 45 mg de taurine dans 1 g d'extrait,
- au plus 45 mg de citrulline dans 1 g d'extrait,
- au plus 2,25 mg d'aminoacides "mycosporine-like" dans 1 g d'extrait,
- au plus 120 ppm de zinc et au plus 60 ppm de sélénium,
- au plus 9 % de floridoside en poids d'extrait.

7. Utilisation selon l'une des revendications 1 à 6 **caractérisée en ce que** les aminoacides "mycosporine-like" sont choisis dans le groupe constitué par la palythine, la shinorine et l'astérina.

8. Utilisation selon l'une des revendications 1 à 7, **caractérisée en ce que** lesdites macroalgues rouges ou lesdits extraits se présentent sous la forme d'une poudre micronisée ou d'une pâte.

9. Composition nutritionnelle, diététique ou nutraceutique, **caractérisée en ce qu'**elle comprend :
- des macroalgues rouges soumises à des conditions extrêmes de température et de luminosité puis récoltées à la fin de l'hiver septentrional, de préférence entre Mars et début Avril, ou des extraits alcooliques, hydroalcooliques ou hydroglycoliques issus de telles algues ;
- des excipients couramment utilisés en nutrition, diététique ou nutraceutique ;
lesdites conditions extrêmes consistant en une exposition à une eau froide comprise entre 0 et 8°C, de salinité comprise entre 10 et 30 pour mille, et contenant des ions nitrate et ammonium et à une intensité lumineuse comprise entre 40 et 120 langley.day⁻¹;
lesdites macroalgues rouges étant choisies parmi les espèces *Grateloupia turuturu, Ahnfeltia plicata, G racilaria sp., Petrocelis middendorfii, Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* et *Chondrus crispus ;*
lesdites macroalgues rouges ou leurs extraits comprenant les constituants suivants :
- le dipeptide citrullinylarginine,
- les aminoacides libres taurine et citrulline et/ou leurs dérivés,
- des aminoacides "mycosporine-like",
- les ions zinc et/ou sélénium,
- le floridoside.

10. Composition selon la revendication 9, **caractérisée en ce que** lesdites macroalgues rouges ou lesdits extraits sont tels que définis dans l'une des revendications 2 à 8.

## Patentansprüche

1. Verwendung von roten Makroalgen, die extremen Temperatur- und Helligkeitsbedingungen ausgesetzt sind, dann am Ende des nördlichen Winters, vorzugsweise zwischen März und Anfang April geerntet werden, oder von alkoholischen, hydroalkoholischen oder hydroglykolischen Extrakten aus solchen Algen, als Antioxidans für die Herstellung einer Ernährungs-, diätetischen oder nutrazeutischen Zusammensetzung,
wobei die extremen Bedingungen darin bestehen, einem kalten Wasser zwischen 0 und 8 °C, das einen Salzgehalt im Bereich zwischen 10 und 30 Promille aufweist und Nitrat- und Ammonium-Ionen enthält, sowie einer Lichtstärke zwischen 40 und 120 langley.day⁻¹ PAR auszusetzen,
wobei die roten Makroalgen aus den Gattungen *Grateloupia turuturu, Ahnfeltia plicata, Gracilaria sp., Petrocelis middendorfii, Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* und *Chondrus crispus* ausgewählt sind,
wobei die roten Makroalgen oder ihre Extrakte die folgenden Bestandteile umfassen:
- das Dipeptid Citrullinylarginin,
- die freien Aminosäuren Taurin und Citrullin und/oder ihre Derivate,
- "mycosporine-like" Aminosäuren,
- die Ionen Zink und/oder Selen,
- Floridosid.

2. Verwendung nach Anspruch 1, **dadurch gekennzeichnet, dass** die roten Makroalgen aus den Gattungen *Grateloupia turuturu, Ahnfeltia plicata, Gracilaria sp., Petrocelis middendorfii, Polyides rotundus, Polysiphonia lanosa, Rhodomela confervoides, Gymnogongrus devoniensis* und *Chondrus crispus* ausgewählt sind.

3. Verwendung nach Anspruch 1 oder 2, **dadurch gekennzeichnet, dass** die roten Makroalgen von der Gattung *Chondrus crispus* sind.

4. Verwendung nach einem der Ansprüche 1 bis 3, **dadurch gekennzeichnet, dass** die extremen Temperatur- und Helligkeitsbedingungen darin bestehen, einem kalten Wasser zwischen 0 und 4 °C, das Nitrat-Ionen mit dem Gehalt von 1 bis 6 µmol und Ammonium-Ionen enthält, auszusetzen.

5. Verwendung nach einem der Ansprüche 1 bis 4, **dadurch gekennzeichnet, dass** die Makroalgen umfassen:
- 2 bis 10 % Citrullinylarginin in Gewicht Trockenmasse,
- 6 bis 30 mg Taurin in 1 g Trockenalge,
- 6 bis 30 mg Citrullin in 1 g Trockenalge,
- 0,7 bis 1,5 mg "mycosporine-like" Aminosäuren in 1 g Trockenalge,
- 20 bis 80 ppm Zink und 5 bis 40 ppm Selen,
- 3 bis 6 % Floridosid in Gewicht Trockenmasse.

6. Verwendung nach einem der Ansprüche 1 bis 4, **dadurch gekennzeichnet, dass** die Extrakte umfassen:
- höchstens 15 % Citrullinylarginin in Gewicht Extrakt,
- höchstens 45 mg Taurin in 1 g Extrakt,
- höchstens 45 mg Citrullin in 1 g Extrakt,
- höchstens 2,25 mg "mycosporine-like" Aminosäuren in 1 g Extrakt,
- höchstens 120 ppm Zink und höchstens 60 ppm Selen,
- höchstens 9 % Floridosid in Gewicht Extrakt.

7. Verwendung nach einem der Ansprüche 1 bis 6, **dadurch gekennzeichnet, dass** die "mycosporine-like" Aminosäuren aus der Gruppe bestehend aus Palythin, Shinorin und Asterina ausgewählt sind.

8. Verwendung nach einem der Ansprüche 1 bis 7, **dadurch gekennzeichnet, dass** die roten Makroalgen oder die Extrakte in Form eines mikronisierten Pulvers oder einer Paste vorliegen.

9. Ernährungs-, diätetische oder nutrazeutische Zusammensetzung, **dadurch gekennzeichnet, dass** sie umfasst:
- roten Makroalgen, die extremen Temperatur- und Helligkeitsbedingungen ausgesetzt sind, dann am Ende des nördlichen Winters, vorzugsweise zwischen März und Anfang April geerntet werden, oder alkoholische, hydroalkoholische oder hydroglykolische Extrakte aus solchen Algen,
- in der Ernährung, Diätetik oder Neutrazeutik üblicherweise verwendete Hilfsstoffe,
wobei die extremen Bedingungen darin bestehen, einem kalten Wasser zwischen 0 und 8°C, das einen Salzgehalt im Bereich zwischen 10 und 30 Promille aufweist und Nitrat- und Ammonium-Ionen enthält, sowie einer Lichtstärke zwischen 40 und 120 langley.day⁻¹ auszusetzen,
wobei die roten Makroalgen aus den Gattungen *Grateloupia turuturu, Ahnfeltia plicata, Gracilaria sp., Petrocelis middendorfii, Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* und *Chondrus crispus* ausgewählt sind,
wobei die roten Makroalgen oder ihre Extrakte die folgenden Bestandteile umfassen:
- das Dipeptid Citrullinylarginin,
- die freien Aminosäuren Taurin und Citrullin und/oder ihre Derivate,
- "mycosporine-like" Aminosäuren,
- die Ionen Zink und/oder Selen,
- Floridosid

10. Zusammensetzung nach Anspruch 9, **dadurch gekennzeichnet, dass** die roten Makroalgen oder die Extrakte so sind wie sie in einem der Ansprüche 2 bis 8 definiert sind.

## Claims

1. Use for the manufacture of a nutritional, dietetic or neutraceutical composition, of red macroalgae subjected to extreme conditions of temperature and luminosity then harvested at the end of the northern winter season, preferably between March and early April, or of alcoholic, hydroalcoholic or hydroglycolic extracts from such algae, as an antioxidant agent;
said extreme conditions consisting of an exposure to cold water of between 0 and 8°C, with a salinity of between 10 and 30 per thousand and containing nitrate and ammonium ions, and to a light intensity of between 40 and 120 langley.day⁻¹ PAR;
said red macroalgae being selected from the species *Grateloupia turuturu, Ahnfeltia plicata, Gracilaria sp., Petrocelis middendorfii*, *Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* and *Chondrus crispus;*
said red macroalgae or their extracts comprising the following constituents:
- citrullinylarginine dipeptide,
- free amino acids taurine and citrulline and/or their derivatives,
- "mycosporine-like" amino acids,
- zinc and/or selenium ions,
- floridoside.

2. Use according to claim 1, **characterized in that** the red macroalgae are selected from the species *Grateloupia turuturu, Ahnfeltia plicata, Gracilaria sp., Petrocelis middendorfii*, *Polyides rotundus, Polysiphonia lanosa, Rhodomela confervoides, Gymnogongrus devoniensis* and *Chondrus crispus*

3. Use according to claim 1 or 2, **characterized in that** the red macroalgae are of the species *Chondrus crispus*

4. Use according to one of claims 1 to 3, **characterized in that** the extreme conditions of temperature and luminosity consist of an exposure to cold water of between 0 and 4°C, and containing nitrate ions with a content of 1 to 6 µmoles, and ammonium ions.

5. Use according to one of claims 1 to 4, **characterized in that** said macroalgae comprise:
- from 2 to 10 % of citrullinylarginine by weight of dry matter,
- from 6 to 30 mg of taurine in 1 g of dry alga,
- from 6 to 30 mg of citrulline in 1 g of dry alga,
- from 0.7 to 1.5 mg of "mycosporine-like" amino acids in 1 g of dry alga,
- from 20 to 80 ppm of zinc and from 5 to 40 ppm of selenium,
- from 3 to 6 % of floridoside by weight of dry matter.

6. Use according to one of claims 1 to 4, **characterized in that** said extracts comprise:
- at most 15% of citrullinylarginine by weight of extract,
- at most 45 mg of taurine in 1 g of extract,
- at most 45 mg of citrulline in 1 g of extract,
- at most 2.25 mg of "mycosporine-like" amino acids in 1 g of extract,
- at most 120 ppm of zinc and at most 60 ppm of selenium,
- at most 9% of floridoside by weight of extract.

7. Use according to one of claims 1 to 6, **characterized in that** the "mycosporine-like" amino acids are selected from the group consisting of palythine, shinorine and asterina.

8. Use according to one of claims 1 to 7, **characterized in that** said red macroalgae or said extracts are in form of a micronized powder or a paste.

9. A nutritional, dietetic or nutraceutical composition, **characterized in that** it comprises:
- red macroalgae subjected to extreme conditions of temperature and luminosity then harvested at the end of the northern winter season, preferably between March and early April, or alcoholic, hydroalcoholic or hydroglycolic extracts from such algae;
- excipients commonly used in nutrition, dietetics or nutraceuticals;
said extreme conditions consisting of an exposure to cold water of between 0 and 8°C, with a salinity of between 10 and 30 per thousand and containing nitrate and ammonium ions, and to a light intensity of between 40 and 120 langley.day⁻¹;
said red macroalgae being selected from the species *Grateloupia turuturu, Ahnfeltia plicata, Gracilaria sp., Petrocelis middendorfii*, *Polyides rotundus, Polysiphonia lanosa, Rhodomela confervoides, Gymnogongrus devoniensis, Callophyllis crassifolia, Callophyllis crenulata, Callophyllis megalocarpa, Callophyllis pinnata* and *Chondrus crispus;*
said red macroalgae or their extracts comprising the following constituents:
- citrullinylarginine dipeptide,
- free amino acids taurine and citrulline and/or their derivatives,
- "mycosporine-like" amino acids,
- zinc and/or selenium ions,
- floridoside.

10. A composition according to claim 9, **characterized in that** said red macroalgae or said extracts are as defined in one of claims 2 to 8.
